# EUROPEAN PATENT SPECIFICATION

(11) **EP 2 835 149 B1**
(45) Date of publication and mention of the grant of the patent: **01.11.2017**
(21) Application number: 13769074.9
(22) Date of filing: 15.03.2013
(51) Int. Cl.: A61N 5/10, G21K 1/04

(54) **COLLIMATOR FOR NEUTRON CAPTURE THERAPY AND NEUTRON CAPTURE THERAPY APPARATUS**
KOLLIMATOR FÜR NEUTRONENEINFANGTHERAPIE UND VORRICHTUNG FÜR NEUTRONENEINFANGTHERAPIE
COLLIMATEUR POUR UNE THÉRAPIE PAR CAPTURE DE NEUTRONS ET APPAREIL DE THÉRAPIE PAR CAPTURE DE NEUTRONS

(30) Priority: 30.03.2012 JP 2012080230
(43) Date of publication of application: 11.02.2015
(73) Proprietor: Sumitomo Heavy Industries, Ltd., Tokyo 141-6025 (JP); Kyoto University, Kyoto-shi, Kyoto 606-8501 (JP)
(72) Inventor: OGASAWARA Tsuyoshi, Niihama-shi Ehime 792-8588 (JP); MARUHASHI Akira, Sennan-gun Osaka 590-0494 (JP)
(74) Representative: Schmidbauer, Andreas Konrad
(86) International application number: PCT/JP2013/057485
(87) International publication number: WO 2013/146373

(56) References cited:
- JP-A- S59 230 199
- JP-A- 2002 186 676
- JP-A- 2005 185 321
- JP-A- 2007 195 877
- US-A- 4 463 266

## Description

### Technical Field

The present invention relates to a collimator for neutron capture therapy and a neutron capture therapy apparatus.

### Background Art

In the related art, as shown in JP 2009-189725 A, a neutron beam irradiation apparatus which irradiates an affected area in the body of a patient with a neutron beam is known. In this apparatus, a neutron beam is generated by irradiating a target with an ion beam (a charged particle beam) and the generated neutron beam is decelerated by a deceleration device and then emitted toward the patient.

This apparatus has a collimator disposed between the deceleration device and the patient. The collimator is a rectangular parallelepiped-shaped member made of a lithium fluoride-containing polyethylene material and a neutron extraction port having a predetermined size is provided at the center thereof. The size of the neutron extraction port is formed in accordance with the shape of an irradiation range for each patient. The neutrons emitted from the deceleration device pass through the neutron extraction port of the collimator, thereby being shaped to a predetermined irradiation range.

If the neutron beam is irradiated to the affected area of the patient, a nuclear reaction of the neutron beam with boron incorporated as a compound into the affected area in advance occurs, whereby α rays (a He beam) and a Li beam which are heavy charged particle beams are produced, and cells of the affected area are destroyed by the particle beams. In this apparatus, a placement stand on which a patient is put and the collimator are provided so as to be movable along a neutron extraction direction. Due to such a configuration, alignment of the neutron extraction port of the collimator with an irradiation target is easily performed, and thus improvement in irradiation accuracy is attained.

US 4,463,266 A was used as a basis for the preamble of claim 1 and discloses a neutron collimator with an adjustable irradiation field for an effective neutron radiation source which includes a protective radiation casing; a frame surrounded by the casing; a plurality of individual carrier arms mounted on the frame; a plurality of pairs of opposite elongated wedge-shaped slabs arranged side by side such that respective ones of the opposite wedge-shaped slabs form a fan-shaped configuration which converges toward an apex at the neutron radiation source, each wedge-shaped slab being mounted for rotational and translational movement on a respective carrier arm such that the wedge-shaped slabs of each pair are mounted for motion towards and away from each other along a path which intersects the irradiation field for the neutron radiation source and such that the inner side surface of each wedge-shaped slab is always directed generally towards the neutron radiation source; and at least one bearing provided between each wedge-shaped slab and the respective carrier arm on which it is movably mounted.

JP S59 230199 A discloses a collimating plate for a neutron ray.

### Summary of Invention

### Technical Problem

A therapeutic method described above is called neutron capture therapy (NCT; Neutron Capture Therapy). Usually, in neutron capture therapy, a neutron dose of neutrons which are irradiated to an affected area is predetermined before the start of treatment. In the apparatus of the related art described above, although improvement in irradiation accuracy is attained, according to a site which is irradiated, a certain gap occurs between the rectangular parallelepiped-shaped collimator and the patient. For this reason, there is a case where the neutrons having passed through the collimator are dispersed before reaching the body surface of the patient. In this case, a neutron dose which is irradiated to tissues other than the affected area increases. Further, a neutron dose which is irradiated to the affected area per hour becomes insufficient, and thus it takes a longer time to irradiate a predetermined neutron dose to the affected area.

The present invention has an object to provide a collimator for neutron capture therapy and a neutron capture therapy apparatus, in which a neutron dose which is irradiated to an affected area per hour is secured, and thus it is possible to shorten irradiation time.

### Solution to Problem

In order to solve the above-described problem, according to an aspect of the present invention, there is provided a collimator for neutron capture therapy which sets an irradiation range of a neutron beam in accordance with an irradiation target in an irradiated body, as set forth in claim 1.

According to the collimator for neutron capture therapy, since at least some leaf plates among the plurality of leaf plates are slidable along the second direction orthogonal to the irradiation direction, it is possible to accurately set the irradiation range with respect to the irradiation target. In addition, since at least some leaf plates are also slidable in the irradiation direction, it is possible to bring marginal edges of the leaf plates close to the surface of the irradiated body so as to conform to the shape of an irradiation site of the irradiated body. A gap between the collimator and the irradiated body is narrowed thereby, and thus dispersion of neutrons is suppressed. As a result, a neutron dose which is irradiated to the affected area per hour is secured, and thus it is possible to shorten irradiation time.

Further, in the collimator for neutron capture therapy described above, at least some leaf plates have a plurality of separate leaf plates divided in the second direction and the plurality of separate leaf plates are configured to be respectively independently slidable in the irradiation direction. In this case, for example, even in a case where the irradiation site of the irradiated body is rounded over the second direction, by sliding the respective separate leaf plates in the irradiation direction, it is possible to bring the marginal edges of the leaf plates close to the surface of the irradiation site. Dispersion of neutrons is even further suppressed thereby.

Preferred embodiments of the present invention may be gathered from the dependent claims.

According to a preferred embodiment of the collimator for neutron capture therapy, since the inner separate leaf plate is also slidable in the irradiation direction, it is possible to bring the marginal edges of the leaf plates close to the surface of the irradiated body so as to conform to the shape of the irradiation site of the irradiated body. A gap between the collimator and the irradiated body is narrowed thereby, and thus dispersion of neutrons is suppressed. As a result, a neutron dose which is irradiated to the affected area per hour is secured, and thus it is possible to shorten irradiation time.

Further, the support portion in the irradiation direction may be a locking portion which is provided at the separate leaf plate adjacent to the separate leaf plate supported so as to be slidable along the irradiation direction and is configured to lock an end portion of the separate leaf plate supported so as to be slidable along the irradiation direction. In this case, since an outer separate leaf plate doubles as the support portion in the irradiation direction, the need to separately prepare a member which supports the inner separate leaf plates is eliminated. Therefore, it is possible to suppress an increase in the size of the entire collimator.

In order to solve the above-described problem, according to still another aspect of the present invention, there is provided a neutron capture therapy apparatus including: a neutron beam generation section which is configured to generate a neutron beam; the above-described collimator for neutron capture therapy; and a collimator supporting section which is configured to support the collimator for neutron capture therapy.

### Advantageous Effects of Invention

According to the collimator for neutron capture therapy and the neutron capture therapy apparatus related to the present invention, a neutron dose which is irradiated to an affected area per hour is secured, and thus it is possible to shorten irradiation time.

### Brief Description of Drawings

Fig. 1 is a side view showing a schematic configuration of a neutron beam irradiation apparatus which is provided with a collimator according to an embodiment of the present invention.
Fig. 2 is a conceptual diagram showing a state where neutrons passing through the collimator in Fig. 1 are irradiated to an irradiation target of an irradiated body.
Fig. 3(a) is a front view of the collimator, and Fig. 3(b) is a side view of the collimator.
Fig. 4(a) is a front view showing an irradiation field formed by leaf plates, and an irradiation target, and Fig. 4(b) is a cross-sectional view taken along line IVB - IVB of Fig. 4(a).
Fig. 5(a) is a cross-sectional view of a collimator according to another embodiment of the present invention, and Fig. 5(b) is a cross-sectional view of a collimator of the related art.

### Description of Embodiments

Hereinafter, embodiments of the present invention will be described with reference to the drawings. In the following description, a neutron beam irradiation apparatus provided with a collimator for neutron capture therapy according to an embodiment will be described.

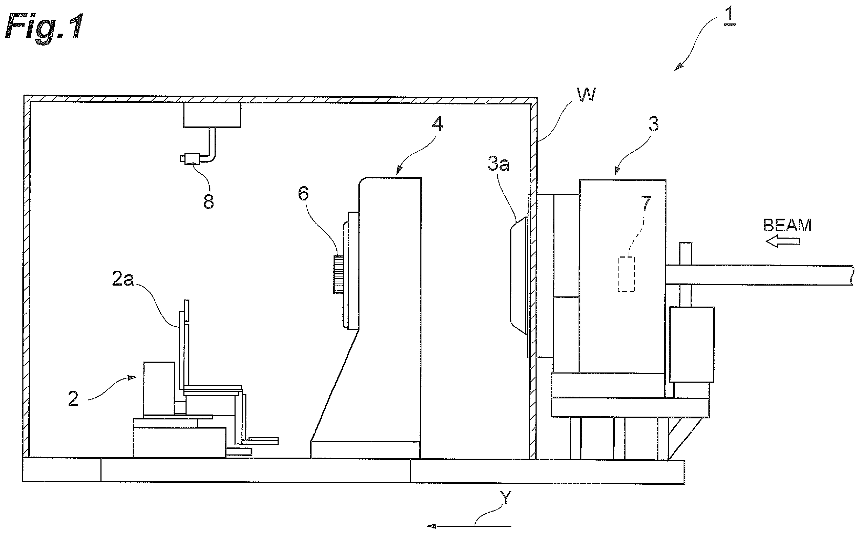

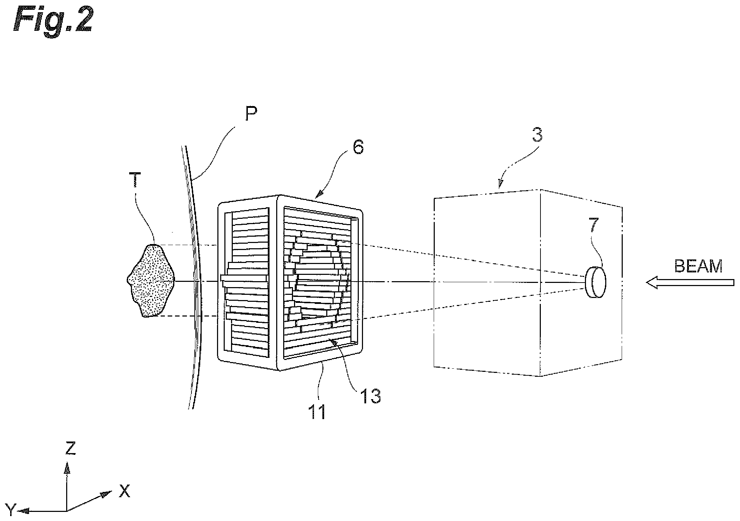

As shown in Figs. 1 and 2, a neutron beam irradiation apparatus 1 is a neutron capture therapy apparatus for irradiating an affected area (an irradiation target) T of a patient (an irradiated body) P with a neutron beam. The neutron beam irradiation apparatus 1 is an apparatus for boron neutron capture therapy (BNCT; Boron Neutron Capture Therapy) which selectively destroys cells of the affected area T by a nuclear reaction of a neutron with boron (10B) incorporated as a compound into the affected area T in advance. The neutron beam irradiation apparatus 1 is provided with a movable stand 2 on which the patient P is placed, a deceleration device 3 which decelerates neutrons, a target (a neutron beam generation section) 7 which generates neutrons, and a collimator stand (a collimator supporting section) 4 provided between the movable stand 2 and the deceleration device 3. Further, the neutron beam irradiation apparatus 1 is provided with a CR system 8 for imaging an X-ray image of the affected area T of the patient P.

The movable stand 2 has a placement stand 2a on which the patient P sits down. The placement stand 2a is configured such that the patient P can also lie down thereon by changing the shape thereof. The movable stand 2 is made so as to be movable in an up-and-down direction (a vertical direction), a left-right direction (a horizontal direction, a direction approaching or being separated from the deceleration device 3), and a front-back direction (a horizontal direction, a direction perpendicular to the left-right direction).

The target 7 generates neutrons by irradiating an ion beam (for example, a proton beam) emitted from an accelerator (not shown) thereto. The deceleration device 3 decelerates neutrons generated by the target 7. The neutrons decelerated by the deceleration device 3 are emitted to the patient P side. A shielding wall W is configured by concrete or the like and prevents unnecessary radiation irradiation to the patient P or the outside of a treatment room. A tip portion 3a of the deceleration device 3 passes through the shielding wall W. The collimator stand 4 moves, thereby approaching the deceleration device 3, whereby the tip portion 3a can enter into the back side of the collimator stand 4. Regarding the collimator stand 4, a "back side" is the upstream side of the ion beam and a "front side" is the patient P side.

In the collimator stand 4, a collimator (a collimator for neutron capture therapy) 6 is provided to protrude to the movable stand 2 side. The collimator 6 is for shaping the neutrons emitted from the deceleration device 3 into a predetermined shape (an irradiation range) and extracting the neutrons in an irradiation direction Y. The collimator stand 4 is made so as to be movable in the front-back direction. In the back surface of the collimator stand 4, a concave portion receiving the tip portion 3a of the deceleration device 3 is formed. The collimator stand 4 is used in a state where the tip portion 3a has entered into the concave portion on the back side. At the time of treatment, the placement stand 2a with the patient P placed thereon is brought close to the front side of the collimator stand 4, whereby the collimator 6 is disposed so as to face the affected area T of the patient P.

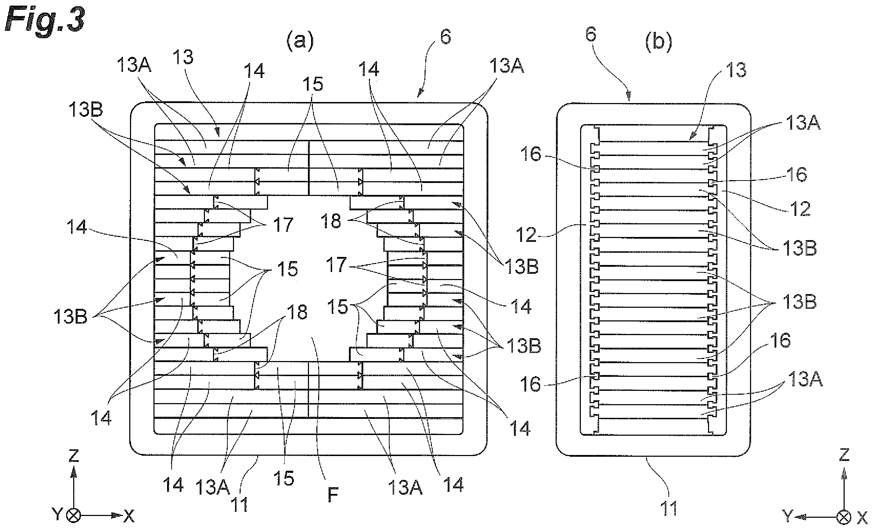

As shown in Figs. 2 and 3, the collimator 6 sets an irradiation field F which is an irradiation range of a neutron beam, in accordance with the domain of the affected area T of the patient P. The collimator 6 has an outer shape of a rectangular parallelepiped shape. The collimator 6 has a plurality of leaf plates 13A and 13B stacked in an up-and-down direction (a first direction) Z orthogonal to the irradiation direction Y. In other words, the plurality of leaf plates 13A and 13B are arranged along a plate thickness direction thereof. In addition, the plurality of leaf plates 13A and 13B may be arranged along a left-right direction X. Each of the leaf plates 13A and 13B is made of lithium fluoride-containing polyethylene and has a rectangular plate shape. The plurality of leaf plates 13A and 13B are retained in a collimator holder 11, thereby configuring a leaf plate group 13.

A guide member (a support portion in a second direction) 12 having a plurality of projections 16 for guiding the leaf plates 13A and 13B is disposed between the collimator holder 11 and the leaf plate group 13. An end portion in the irradiation direction Y of each of the leaf plates 13A and 13B, which comes into contact with the guide member 12, has a concavo-convex shape capable of being fitted to the projection 16 of the guide member 12.

In the collimator 6 of this embodiment, each of the leaf plates 13A (in the example of Fig. 3, four for each of the top and the bottom) disposed at an upper end portion and a lower end portion of the leaf plate group 13 is a plate material divided into two in the left-right direction X. Each of the leaf plates 13B disposed at an intermediate portion excluding the upper end portion and the lower end portion of the leaf plate group 13 is configured by two separate leaf plates 14 and 15 per one side divided in the left-right direction (the second direction) X. More specifically, the separate leaf plate 14 and the separate leaf plate 15 are provided side by side in the left-right direction X. The separate leaf plate 14 is disposed outside (that is, near the collimator holder 11), and the separate leaf plate 15 is disposed inside (that is, near the irradiation field F) adjacent to the separate leaf plate 14. The separate leaf plate 14 and the separate leaf plate 15 are made so as to be slidable in the left-right direction X in an integrated manner. In this manner, the leaf plate 13B is slidable in the left-right direction X, and therefore, it is possible to arbitrarily set the shape and the size of the irradiation field F. In addition, the leaf plate 13A is not limited to a case of being divided into two in the left-right direction X and may be divided into, for example, four.

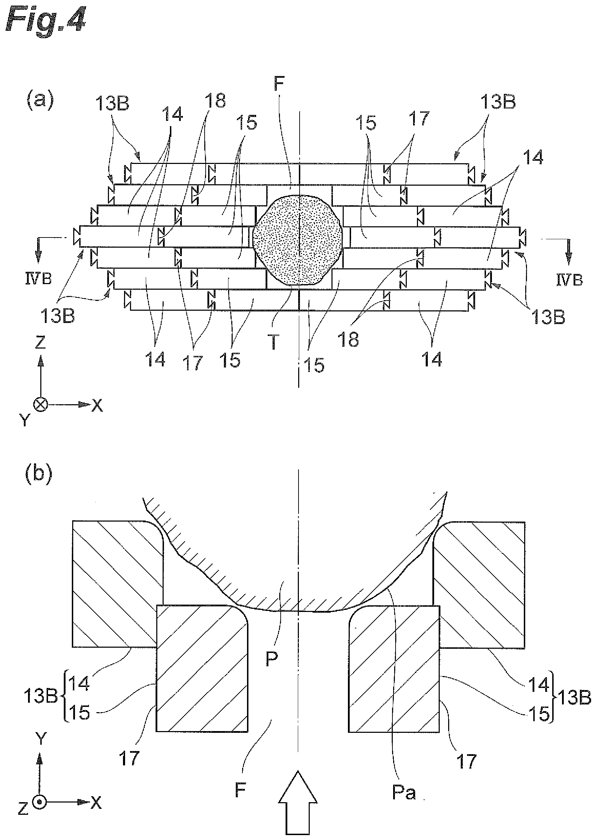

In addition, in the collimator 6, the separate leaf plate 15 of the leaf plate 13B is also made so as to be slidable in the irradiation direction Y. As shown in Figs. 3 and 4, a protruding portion 17 protruding in the left-right direction X and also extending in the irradiation direction Y is formed on the end face of the separate leaf plate 15, which comes into contact with the separate leaf plate 14. On the other hand, a groove portion (a support portion in the irradiation direction) 18 extending in the irradiation direction Y is formed in the end face of the separate leaf plate 14, which comes into contact with the separate leaf plate 15. The separate leaf plate 14 and the separate leaf plate 15 are respectively made so as to be slidable in the irradiation direction Y in a state where the protruding portion 17 is fitted into the groove portion 18. In addition, the groove portion 18 may also function as a locking portion which locks an end portion of the separate leaf plate 15 supported so as to be slidable in the irradiation direction Y.

The collimator 6 having the above-described configuration is a 2-axis slide type multileaf collimator slidable in the left-right direction X and the irradiation direction Y. In the collimator 6, it is possible to freely change the outer shape of the leaf plate group 13 in the left-right direction X and the irradiation direction Y.

When treating the affected area T of the patient P, the shape of an irradiation site is examined in advance and the position of each of the leaf plates 13A and 13B is adjusted so as to conform to the shape. The position of each of the leaf plates 13A and 13B is manually adjusted by a handler. Then, an irradiation site of the patient P is brought close to the collimator 6 in which the position adjustment has been completed, and a neutron beam is irradiated toward the affected area T with a predetermined neutron dose.

According to the collimator 6 of this embodiment, the plurality of leaf plates 13B are slidable along the left-right direction X orthogonal to the irradiation direction Y, and therefore, it is possible to accurately set the irradiation field F with respect to the affected area T. In addition, the separate leaf plate 15 of the leaf plate 13B is also slidable in the irradiation direction Y, and therefore, it is possible to bring a marginal edge of the separate leaf plate 15 close to a body surface Pa of the patient P so as to conform to the shape of the irradiation site. A gap between the collimator 6 and the patient P is narrowed thereby, and thus dispersion of neutrons is suppressed. As a result, a neutron dose which is irradiated to the affected area T per hour is secured, and thus it is possible to shorten irradiation time. This also contributes to a reduction in the burden on the patient P at the time of treatment.

A neutron has a property of being relatively easily dispersed. In a collimator of the related art, since it is not possible to change an outer shape, it is difficult to make an end face of the collimator be fitted according to the shape of an irradiation site. According to the collimator 6 of this embodiment, a gap between the body surface Pa of the patient P and the leaf plates 13A and 13B is made as small as possible, and thus it is possible to block dispersion of neutrons. In this way, it is possible to attain the optimization of a neutron dose.

Further, the plurality of separate leaf plates 15 of the leaf plates 13B are respectively independently slidable in the irradiation direction Y, and therefore, for example, even in a case where the irradiation site of the patient P is rounded over the left-right direction X, by sliding the respective separate leaf plates 14 and 15 in the irradiation direction Y, it is possible to bring the marginal edges of the leaf plates 14 and 15 close to the body surface Pa of the irradiation site (refer to Fig. 4). Dispersion of neutrons is even further suppressed thereby.

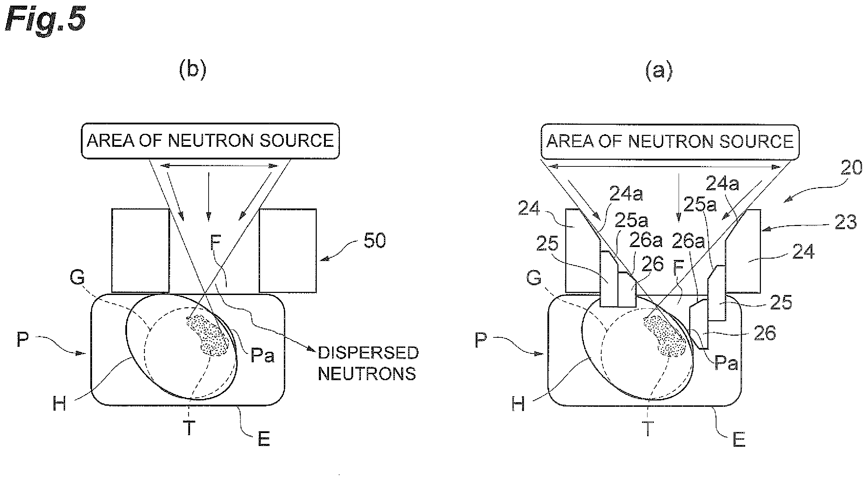

Fig. 5(a) is a cross-sectional view of a collimator according to another embodiment, and Fig. 5(b) is a cross-sectional view of a collimator of the related art. A leaf plate 23 of a collimator 20 shown in Fig. 5(a) is different from the leaf plate 13B of the collimator 6 shown in Fig. 3 in that the leaf plate 23 is provided with separate leaf plates 24, 25, and 26 configured so as to be three pieces with respect to one side and that inner corner portions on the side opposite to the patient P of the respective separate leaf plates 24 to 26 are cut out, whereby tapered portions 24a to 26a are formed at the respective separate leaf plates 24 to 26. Among the separate leaf plates 24, 25, and 26, the separate leaf plates 25 and 26 with the exception of the separate leaf plate 24 are made so as to be slidable in the irradiation direction Y.

As shown in Fig. 5(a), due to the points that the respective separate leaf plates 24 to 26 have the tapered portions 24a to 26a and that the separate leaf plates 25 and 26 slide in the irradiation direction Y, it is possible to make a direction in which neutrons are incident wider as a whole. In Fig. 5(a), a case where a neutron beam is irradiated to the affected area T (for example, a tumor) which is present in a neck portion G of the patient P is shown. The neck portion G of the patient P is present between a jaw portion H and a shoulder portion E. Even in such a case, the separate leaf plates 25 and 26 slide so as to come into close contact with the body surface Pa of the patient P, whereby it is possible to prevent the dispersion of neutrons from a gap between a body surface and the collimator 20. Due to the effects, high-dose-rate irradiation becomes possible, and thus the shortening of irradiation time is attained. Furthermore, due to having a tapered structure obliquely cut on the neutron incidence side, it is possible to widen a direction of incident neutrons, and thus it is possible to use a lot of neutrons capable of contributing to the irradiation to the affected area T.

On the other hand, in a collimator 50 of the related art shown in Fig. 5(b), neutron are dispersed from a gap between a leaf plate and the body surface Pa of the patient P, and thus a neutron beam source is wasted.

The basic role of a collimator is for cutting radiation outside an irradiated area, thereby making exposure of normal tissue be less than or equal to a negligible amount. Radiation therapy (for example, X-ray therapy or proton therapy) of the related art basically collimates radiation which is emitted from one point and goes straight ahead, in accordance with an irradiated area, and a flat plate-shaped collimator is set at a place distant by several cm from an irradiated body surface. In a case of neutrons, most thereof become scattered radiation even in an atomic reactor or even in an accelerator and neutrons arriving in a direction of a collimator from a very large area compared to the collimator are used. In a case of neutrons, due to being a volume source, rather than a point source, an angle (a solid angle) over which a collimator opening can be viewed from a tumor is large, and thus the availability of neutrons from a wider radiation source area becomes a condition for increasing a dose rate. At the same time, if dispersion of neutrons from the gap between the collimator and the irradiated body surface can be prevented, this is reflected in an increase in the dose rate of the component, and it is possible to avoid portions other than an irradiation area being exposed to dispersed neutrons. A rise in the utilization efficiency of the neutrons is one effect of the neutron collimators 6 and 20 according to the embodiments of the present invention. Other effects are to reduce the exposure of normal tissue by cutting radiation outside an irradiated area and to attain the optimization of irradiation taking into account the correlation between reduction measures and a request of a conflicting dose rate increase.

The embodiments of the present invention have been described above. However, the present invention is not limited to the above-described embodiments.

For example, the separate leaf plates are not limited to two or three and there may be four or more. In a case where there are three or more separate leaf plates, it is possible to make the separate leaf plates other than the separate leaf plates on the outermost side (the collimator holder 11 side in the left-right direction X) be slidable in the irradiation direction Y. In addition, only the separate leaf plates on the innermost side (the irradiation field F side) may be made to be slidable in the irradiation direction Y. Further, a drive mechanism connected to each of the separate leaf plates 14 and 15 of the leaf plate 13A and the leaf plate 13B may be provided. As the drive mechanism, for example, a roller and a motor, or a piston or the like can be used. Due to the drive mechanism, it is possible to automate the movement and the positioning of the leaf plates 13A and 13B.

The support portion in the irradiation direction may be the groove portion 18 provided at the separate leaf plate 14 and a support mechanism may be provided as a separate member at an end portion of the separate leaf plate 14. The collimator stand 4 may not be provided independently, and a portion supporting the collimator may be provided at, for example, the shielding wall W or the deceleration device 3.

Furthermore, the present invention is not limited to BNCT using a boron compound and may be applied to NCT using other elements. For example, the present invention can also be applied as a collimator which is used in a therapeutic method using a nuclear reaction of a neutron with gadolinium (157Gd or the like). In a case where the speed of a neutron beam obtained from the neutron beam generation section is a speed suitable for treatment, the deceleration device is unnecessary.

### Industrial Applicability

The present invention is applicable to a collimator for neutron capture therapy and a neutron capture therapy apparatus, in which a neutron dose which is irradiated to an affected area per hour is secured, and thus it is possible to shorten irradiation time.

1: neutron beam irradiation apparatus
6, 20: collimator
13A, 13B: leaf plate
14, 15: separate leaf plate
18: groove portion (support portion in an irradiation direction)
F: irradiation field (irradiation range)
P: patient (irradiated body)
T: affected area (irradiation target)
X: left-right direction (second direction)
Y: irradiation direction
Z: up-and-down direction (first direction)

## Claims

1. A collimator (6) for neutron capture therapy which sets an irradiation range of a neutron beam in accordance with an irradiation target (T) in an irradiated body (P), comprising:
a plurality of leaf plates (13A, 13B) stacked in a first direction orthogonal to an irradiation direction of the neutron beam,
wherein at least some leaf plates (14, 15) among the plurality of leaf plates (13A, 13B) are configured to be slidable along a second direction (X) orthogonal to the irradiation direction (Y) and orthogonal to the first direction (Z) and also slidable in the irradiation direction (Y);
**characterized in that**
at least some leaf plates (13A, 13B) have a plurality of separate leaf plates (14, 15) divided in the second direction (X), and
the plurality of separate leaf plates (14, 15) are configured to be respectively independently slidable in the irradiation direction (Y).

2. The collimator (6) for neutron capture therapy according to claim 1,
wherein at least some of the plurality of leaf plates (14, 15) are divided into at least four separate leaf plates (14, 15) in a second direction (Z) orthogonal to the irradiation direction (Y) and the first direction (X);
the collimator (6) further comprising:
a support portion (17) in the second direction (Z) which is configured to support the leaf plate (14) so as to be slidable along the second direction (Z); and
a support portion (18) in the irradiation direction (Y) which is configured to support an inner separate leaf plate (15) of the separate leaf plates (14, 15) divided in the second direction (Z) so as to be slidable along the irradiation direction (Y).

3. The collimator (6) for neutron capture therapy according to Claim 2, wherein the support portion (18) in the irradiation direction (Z) is a locking portion which is provided at the separate leaf plate (15) adjacent to the separate leaf plate (14) supported so as to be slidable along the irradiation direction (Y) and is configured to lock an end portion of the separate leaf plate (14) supported so as to be slidable along the irradiation direction (Y).

4. A neutron capture therapy apparatus (1) comprising:
a neutron beam generation section (7) which is configured to generate a neutron beam;
the collimator (6) for neutron capture therapy according to any one of Claims 1 to 3; and
a collimator supporting section (4) which is configured to support the collimator (6) for neutron capture therapy.

## Patentansprüche

1. Kollimator (6) für die Neutroneneinfangtherapie, der einen Ausstrahlungsbereich eines Neutronenstrahls gemäß einem Bestrahlungsziel (T) in einem Bestrahlungskörper (P) einstellt, der Folgendes aufweist:
eine Vielzahl von Blattplatten (13A, 13B), die in einer ersten Richtung orthogonal zu einer Ausstrahlungsrichtung des Neutronenstrahls gestapelt sind,
wobei zumindest einige Blattplatten (14, 15) innerhalb der Vielzahl von Blattplatten (13A, 13B) konfiguriert sind, um entlang einer zweiten Richtung (X) verschiebbar zu sein, die orthogonal zu der Ausstrahlungsrichtung (Y) und orthogonal zu der ersten Richtung (Z) ist, und ebenfalls in der Ausstrahlungsrichtung (Y) verschiebbar ist;
**dadurch gekennzeichnet, dass**
zumindest einige Blattplatten (13A, 13B) eine Vielzahl von separaten Blattplatten (14, 15) aufweist, die in der zweiten Richtung (X) geteilt sind, und
die Vielzahl der separaten Blattplatten (14, 15) so konfiguriert ist, dass sie jeweils in unabhängiger Weise in der Ausstrahlungsrichtung (Y) verschiebbar sind.

2. Kollimator (6) zur Neutroneneinfangtherapie gemäß Anspruch 1,
wobei zumindest einige der Vielzahl von Blattplatten (14, 15) in zumindest vier separate Blattplatten (14, 15) in einer zweiten Richtung (Z) orthogonal zu der Ausstrahlungsrichtung (Y) und der ersten Richtung (X) unterteilt sind; wobei der Kollimator (6) ferner Folgendes aufweist:
einen Trägerteil (17) in der zweiten Richtung (Z), der konfiguriert ist, um die Blattplatte (14) so zu tragen, dass sie entlang der zweiten Richtung (Z) verschiebbar ist; und
einen Trägerteil (18) in der Ausstrahlungsrichtung (Y), der konfiguriert ist, um eine innere, separate Blattplatten (15) der separaten Blattplatten (14, 15) zu tragen bzw. zu stützen, der in der zweiten Richtung (Z) unterteilt ist, um entlang der Ausstrahlungsrichtung (Y) verschiebbar zu sein.

3. Kollimator (6) zur Neutroneneinfangtherapie gemäß Anspruch 2, wobei der Trägerteil (18) in der Ausstrahlungsrichtung (Z) ein Verriegelungsteil ist, der bei der separaten Blattplatte (15) benachbart zu dem separaten Blattplatte (14) vorgesehen ist, der so getragen wird, dass er entlang der Ausstrahlungsrichtung (Y) verschiebbar ist und konfiguriert ist, um einen Endteil der separaten Blattplatte (14) zu verriegeln, der so gestützt wird, dass er entlang der Ausstrahlungsrichtung (Y) verschiebbar ist.

4. Neutroneneinfangtherapievorrichtung (1), die Folgendes aufweist:
einen Neutronenstrahlerzeugungsabschnitt (7), der konfiguriert ist, um einen Neutronenstrahl zu erzeugen;
den Kollimator (6) für die Neutroneneinfangtherapie gemäß einem der Ansprüche 1 bis 3; und
einen Kollimatorstützteil (4), der konfiguriert ist, um den Kollimator (6) für die Neutroneneinfangtherapie zu tragen bzw. zu stützen.

## Revendications

1. Collimateur (6) pour une thérapie par capture de neutrons, qui règle une plage d'irradiation d'un faisceau de neutrons en fonction d'une cible d'irradiation (T) dans un corps irradié (P), comprenant :
une pluralité de plaques en forme de feuilles (13A, 13B) empilées dans une première direction orthogonale à une direction d'irradiation du faisceau de neutrons,
dans lequel au moins certaines plaques en forme de feuilles (14, 15) parmi la pluralité de plaques en forme de feuilles (13A, 13B) sont agencées pour pouvoir coulisser suivant une deuxième direction (X) orthogonale à la direction d'irradiation (Y) et orthogonale à la première direction (Z) et pouvant aussi coulisser dans la direction d'irradiation (Y) ;
**caractérisé en ce que**
au moins certaines plaques en forme de feuilles (13A, 13B) comportent une pluralité de plaques en forme de feuilles séparées (14, 15) divisées dans la deuxième direction (X), et
la pluralité de plaques en forme de feuilles séparées (14, 15) est agencée pour pouvoir coulisser indépendamment respectivement dans la direction d'irradiation (Y).

2. Collimateur (6) pour une thérapie par capture de neutrons selon la revendication 1,
dans lequel au moins certaines de la pluralité de plaques en forme de feuilles (14, 15) sont divisées en au moins quatre plaques en forme de feuilles séparées (14, 15) dans une deuxième direction (Z) orthogonale à la direction d'irradiation (Y) et à la première direction (X) ;
le collimateur (6) comprenant en outre :
une portion support (17) dans la deuxième direction (Z) qui est agencée pour supporter la plaque en forme de feuille (14) de façon à pouvoir coulisser suivant la deuxième direction (Z) ; et
une portion support (18) dans la direction d'irradiation (Y), qui est agencée pour supporter une plaque en forme de feuille séparée intérieure (15) des plaques en forme de feuilles séparées (14, 15) divisées dans la deuxième direction (Z) de façon à pouvoir coulisser suivant la direction d'irradiation (Y).

3. Collimateur (6) pour une thérapie par capture de neutrons selon la revendication 2, dans lequel la portion support (18) dans la direction d'irradiation (Z) est une portion de verrouillage qui est prévue au niveau de la plaque en forme de feuille séparée (15) adjacente à la plaque en forme de feuille séparée (14) supportée de manière à pouvoir coulisser suivant la direction d'irradiation (Y) et est agencée pour verrouiller une portion d'extrémité de la plaque en forme de feuille séparée (14) supportée de façon à pouvoir coulisser suivant la direction d'irradiation (Y).

4. Appareil de thérapie par capture de neutrons (1) comprenant :
une section de génération de faisceau de neutrons (7) qui est agencée pour générer un faisceau de neutrons ;
le collimateur (6) pour une thérapie par capture de neutrons selon l'une quelconque des revendications 1 à 3 ; et
une section de support de collimateur (4) qui est agencée pour supporter le collimateur (6) pour la thérapie par capture de neutrons.
